**Europäisches Patentamt**

**European Patent Office**

**Office européen des brevets**

(19)

(11) Veröffentlichungsnummer : **0 028 377**
**B1**

(12) **EUROPÄISCHE PATENTSCHRIFT**

(45) Veröffentlichungstag der Patentschrift :
22.09.82

(21) Anmeldenummer : 80106538.4

(22) Anmeldetag : 29.10.80

(51) Int. Cl.³ : **C 07 C 41/06, C 07 C 2/28,**
**C 07 C 43/04, C 10 L 1/02**

(54) Verfahren zur Herstellung eines Gemisches, bestehend im wesentlichen aus Iso-Buten-Oligomeren und Methyl-tert.-butyl-ether, seine Verwendung und Treibstoffe, enthaltend ein solches Gemisch.

(30) Priorität : 03.11.79 DE 2944457

(43) Veröffentlichungstag der Anmeldung :
13.05.81 (Patentblatt 81/19)

(45) Bekanntmachung des Hinweises auf die Patenterteilung : 22.09.82 Patentblatt 82/38

(84) Benannte Vertragsstaaten :
BE DE FR IT NL

(56) Entgegenhaltungen :
DE B 1 213 837
GB A 907 429
US A 2 480 940

(73) Patentinhaber : EC ERDÖLCHEMIE GMBH
Postfach 75 2002
D-5000 Köln 71 (DE)

(72) Erfinder : Schleppinghoff, Bernhard, Dr.
Adolf-Kolping-Strasse 5
D-4047 Dormagen 1 (DE)

(74) Vertreter : Dill, Erwin, Dr. et al
c/o BAYER AG Zentralbereich Patente Marken und
Lizenzen Bayerwerk
D-5090 Leverkusen 1 (DE)

EP 0 028 377 B1

# 0 028 377

### Verfahren zur Herstellung eines Gemisches, bestehend im wesentlichen aus iso-Buten-Oligomeren und Methyl-tert.-butyl-ether, seine Verwendung und Treibstoffe, enthaltend ein solches Gemisch

Die vorliegende Erfindung betrifft ein Verfahren zur Herstellung eines Gemisches, das im wesentlichen aus iso-Buten-Oligomeren und Methyl-tert.-butyl-ether besteht, aus iso-Buten-haltigen $C_4$-Schnitten, die Verwendung eines solchen Gemisches als Treibstoff oder Zusatz zu einem Treibstoff mit hoher Oktanzahl und Treibstoffe für Vergasermotoren mit hoher Oktanzahl, die ein solches Gemisch enthalten.

Beim thermischen und katalytischen Cracken von Mineralölfraktionen, beispielsweise Leichtbenzingemischen, sowie bei der Dehydrierung von n- und iso-Butan, fallen nach der Entfernung von Butadien $C_4$-Schnitte an, die neben n-, iso-Butan und n-Butenen größere Mengen iso-Buten enthalten. Dieses iso-Buten kann einer vielfältigen Verwendung zugeführt werden und ist u.a. Ausgangsprodukt für die Herstellung von Alkylatbenzinen, Di- und Triisobuten (DIB und TIB) sowie für Methyl-tert.-butyl-ether (MTBE). Diese Produkte haben neben anderem eine große Bedeutung als Oktanzahlverbesserer für Motortreibstoffe. Die Herstellung dieser Stoffe geschieht gewöhnlich an sauren Katalysatoren, wie Schwefelsäure, Phosphorsäure, deren Anhydriden oder an sauren Ionenaustauschern und ist seit langem bekannt.

So entsteht beim Erhitzen eines $C_4$-Schnittes mit 50 bis 70 Gew.-%iger Schwefelsäure auf 85 bis 140 °C hauptsächlich Diisobuten (US-Patent 2 237 292).

Ebenso entsteht hauptsächlich Diisobuten beim Behandeln von iso-Buten in iso-Butan bei 0 °C mit Phosphorpentoxid, wobei bei höherer Temperatur die Bildung von Triisobuten bevorzugt wird (US-Patent 2 409 727).

Neuere Verfahren beschreiben die Umsetzung von iso-Buten zu Diisobuten ohne Anwendung von Schwefelsäure z.B. in Gegenwart von sauren Ionenaustauschern, wobei die Reaktion in mehreren hintereinandergeschalteten Schleifenreaktoren oder in einem Röhrenreaktor durchgeführt werden kann (Hydrocarbon Proc., *Vol. 52* (4), 171 (1973)).

Die bisher bekannten Verfahren zur Erzeugung von Di-isobuten und Triisobuten haben jedoch den Nachteil, daß sie bei Verwendung von Säuren und deren Anhydriden große Korrosions- und Abfallbeseitigungsprobleme mit sich bringen und bei der Verwendung beispielsweise von Ionenaustauschern in Schleifenreaktoren Probleme bei der Entfernung des Katalysators auftreten. Außerdem führt bei weitgehender Umsetzung des i-Butens die lange Verweilzeit in Schleifen- und Röhrenreaktoren zur unerwünschten Bildung von höheren Oligomeren und zu starker Isomerisierung des wertvollen Buten-1 zu Buten-2.

Setzt man iso-Buten oder einen iso-Buten-haltigen $C_4$-Schnitt am sauren Ionenaustauscher mit Methanol um, so erhält man Methyl-tert.-butyl-ether. Um eine gute Selektivität bei der Umsetzung zu erreichen, wird Methanol im Überschuß zugegeben. Nach der Reaktion werden die nicht umgesetzten $C_4$-Kohlenwasserstoffe, das nicht umgesetzte Methanol und der gebildete Ether in einer Destillation voneinander getrennt (Hydrocarbon Proc., *Vol. 56* (11), 185 (1977)).

Hierbei erhält man zwar ein einheitliches Produkt, jedoch ist für den MTBE als Mischkomponente zum Vergaserkraftstoff infolge seines niedrigen Siedepunktes die Zumischung höher siedender Komponenten, wie aromatenhaltige Reformatschnitte, notwendig. Weiter ist bekannt, $C_4$-Gemische zunächst einer Verätherung mit Methanol zu unterwerfen, wobei der überwiegende Anteil des iso-Butens zu Methyl-tert.-butyl-ether umgesetzt wird, und den Rest einer Alkylierungsreaktion zu unterwerfen, um so ein hochoktaniges MTBE/$C^8$-Kohlenwasserstoffgemisch zu erhalten (DE-OS 2 246 004). Das genannte Verfahren zur Herstellung eines Gemisches aus MTBE und $C^8$-Kohlenwasserstoffen zum direkten Einsatz als Vergaserkraftstoff hat die folgende Nachteile : Nach der Veretherung müssen die $C_4$-Kohlenwasserstoffe, der MTBE und die höheren Kohlenwasserstoffe sorgfältig getrennt werden. Weiterhin müssen die verbliebenen $C_4$-Kohlenwasserstoffe vor der Alkylierung durch eine Wasserwäsche vom Methanol befreit werden. Der MTBE muß ebenfalls durch eine Wasserwäsche vom überschüssigen Methanol getrennt werden. Darüber hinaus erfolgt die Alkylierungsreaktion des genannten Verfahrens in Gegenwart von Schwefelsäure, womit die bekannten Entsorgungsprobleme für die Beseitigung der Abfallschwefelsäure und die bekannten Korrosionsprobleme auftreten.

Es wurde nun ein Verfahren zur Herstellung eines Gemisches, das im wesentlichen aus iso-Buten-Oligomeren und Methyl-tert.-butyl-ether besteht, gefunden, daß dadurch gekennzeichnet ist, daß man

a) ein Gemisch der geradkettigen und verzweigten Butane und Butene an einem sauren Kontakt so umsetzt, daß 50 bis 90 Gew.-% des in dem Gemisch enthaltenen iso-Butens in iso-Buten-Oligomere umgewandelt werden,

b) das so erhaltene Umsetzungsgemisch anschließend mit einer mindestens molaren Menge Methanol, bezogen auf das restliche iso-Buten, an einem sauren Kontakt umsetzt und

c) die nicht umgesetzten Butane und Butene vom Reaktionsgemisch abtrennt.

Als das Gemisch der geradkettigen und verzweigten Butane und Butene für das erfindungsgemäße Verfahren sei beispielsweise ein $C_4$-Schnitt, wie er etwa beim thermischen oder katalytischen Cracken von Mineralölfraktionen anfällt, nachdem das darin enthaltene Butadien entfernt worden ist, genannt. Die typische Zusammensetzung solcher $C_4$-Schnitte ist in der folgenden Tabelle aufgeführt :

| | Steam Cracker | | Cat Cracker | |
|---|---|---|---|---|
| n-Butan | 6-8 | Gew.-% | etwa 10 Gew.-% | |
| i-Butan | 2-3 | Gew.-% | etwa 34 Gew.-% | |
| i-Buten | 44-49 | Gew.-% | etwa 15 Gew.-% | |
| Buten-1 | 24-28 | Gew.-% | etwa 13 Gew.-% | |
| Buten-2 | 19-21 | Gew.-% | etwa 28 Gew.-% | |

Als saure Kontakte für die Stufen a) und b) des erfindungsgemäßen Verfahrens seien beispielsweise starke Mineralsäuren oder deren Anhydride, wie Schwefelsäure, Phosphorsäure, Schwefeltrioxid, Oleum oder Phosphorpentoxid, sowie stark saure Ionenaustauscher, genannt.

Als stark saure Ionenaustauscher seien beispielsweise sulfonsäuregruppenhaltige Formaldehyd-Phenol-Harze oder sulfonsäuregruppenhaltige mit Divinylbenzol vernetzte Polystyrole genannt.

Es ist möglich, die Stufen a) und b) des erfindungsgemäßen Verfahrens in Gegenwart des gleichen sauren Kontakts oder verschiedener saurer Kontakte durchzuführen.

Bevorzugt für das erfindungsgemäße Verfahren werden stark saure Ionenaustauscher, besonders bevorzugt sulfonsäuregruppenhaltiges, mit Divinylbenzol vernetztes Polystyrol, eingesetzt.

Als Konzentration des sauren Kontaktes für die Stufen a) und b) des erfindungsgemäßen Verfahrens wird die volumetrische Stunden-Raumgeschwindigkeit in Liter $C_4$-Kohlenwasserstoff-Einsatz pro Liter saurer Kontakt pro Stunde angegeben. Hierfür seien beispielsweise Werte von 0,05 bis 50, bevorzugt von 0,2 bis 15, genannt. Die Konzentration des sauren Kontaktes in den Stufen a) und b) kann gleich oder verschieden sein.

In der Stufe a) des erfindungsgemäßen Verfahrens werden beispielsweise 50 bis 90 Gew.-%, bevorzugt 70 bis 80 Gew.-%, des im Ausgangsprodukt enthaltenen iso-Butens in Oligomere umgewandelt.

In der Stufe b) des erfindungsgemäßen Verfahrens wird zur Erzielung eines möglichst weitgehenden Umsatzes des iso-Butens eine mindestens molare Menge Methanol, bezogen auf das restliche iso-Buten, eingesetzt. Bevorzugt wird eine Menge von 1 bis 2 Mol Methanol, besonders bevorzugt 1,2 bis 1,5 Mol Methanol, bezogen auf das restliche iso-Buten, eingesetzt.

Als Temperatur für die Stufe a) des erfindungsgemäßen Verfahrens sei beispielsweise der Bereich von 20 bis 120 °C, bevorzugt von 25 bis 70 °C, genannt.

Außerdem wird bei dem erfindungsgemäßen Verfahren innerhalb dieses Temperaturbereiches die Isomerisierung des n-Buten-1 zu n-Buten-2 weitgehend zurückgedrängt.

Als Temperatur für die Stufe b) des erfindungsgemäßen Verfahrens sei beispielsweise ein Bereich von 25 bis 90 °C, bevorzugt von 45 bis 75 °C, genannt.

Der Druck für die Stufen a) und b) wird so gewählt, daß die am erfindungsgemäßen Verfahren beteiligten Stoffe in flüssiger Form vorliegen. Als Druckbereich sei allgemein der Bereich von 2 bis 30 bar genannt. Selbstverständlich ist der Druck in der dem Fachmann bekannten Weise von der Reaktionsführung im erfindungsgemäßen Verfahren abhängig.

In der Stufe c) des erfindungsgemäßen Verfahrens wird das Reaktionsprodukt zunächst entspannt und gelangt dann in eine dem Fachmann als Debutanizer bekannte Trennapparatur, in der die gesamten $C_4$-Kohlenwasserstoffe als Kopfprodukt entfernt werden. Dieses Kopfprodukt hat einen iso-Butengehalt von unter 1 Gew.-%. Das Sumpfprodukt hat einen Anteil an $C_4$-Kohlenwasserstoffen, der kleiner als 1 % ist und einen Methanol-Gehalt von weniger als 5 %. Es kann beispielsweise als hochoktaniges fertiges Treibstoffgemisch für Vergasermotoren oder als Oktanzahl-verbessernder Zusatz zu Treibstoffen für Vergasermotoren eingesetzt werden. Das erfindungsgemäß hergestellte Gemisch hat folgende Zusammensetzung und folgende Eigenschaften.

| Zusammensetzung und Eigenschaften des Sumpfproduktes | | |
|---|---|---|
| $C_4$-KWS | 1 | Gew.-% |
| $C_8$-KWS | 40-60 | Gew.-% |
| $C_{12}$-KWS | 15-20 | Gew.-% |
| $C_{16}$-KWS | 1-3 | Gew.-% |
| MTBE | 10-30 | Gew.-% |
| Methanol | 0,1-4 | Gew.-% |
| Bromzahl (g$Br_2$/100 ml) | 100 | |
| Siedebeginn | 40 °C | |
| 50 % | 110 °C | |
| Siedeende | 200 °C | |

**0 028 377**

| Dampfdruck nach Reid 0,8 Kp/cm² bei 38 °C | | |
|---|---|---|
| ROZ* | clear | 101-103 |
| ROZ | 0,15 TEL/l*** | 102-103 |
| MOZ** | clear | 83-86 |
| MOZ | 0,15 TEL/l*** | 85-88 |

\* Research-(Test-) Oktanzahl,
\*\* Motor-Oktanzahl,
\*\*\* tetraethyl lead, Bleitetraethyl.

Es kann wünschenswert sein, durch partielle Hydrierung den Olefingehalt des Produktes zu senken. Nach der Hydrierung von ca. 50 % der Olefine erhält man folgende motorische Daten :

| ROZ | clear | 101-103 |
|---|---|---|
| ROZ | 0,15 TEL | 102-105 |
| MOZ | clear | 85-88 |
| MOZ | 0,15 TEL | 88-91 |

Die Bromzahl des partiell hydrierten Produktes ist ungefähr 40.

Die Stufen a) und b) des erfindungsgemäßen Verfahrens können in einem kombinierten Apparat oder in 2 getrennten Apparaten durchgeführt werden. Bei der Anwendung flüssiger saurer Kontakte werden die Apparate vom Ausgangsprodukt im allgemeinen von unten nach oben durchfahren, so daß das spezifisch leichtere Kohlenwasserstoffgemisch durch den spezifisch schwereren Kontakt strömt. Bei der bevorzugten Anwendung fester saurer Kontakte, beispielsweise stark saurer Ionenaustauscher, können die Apparate sowohl von unten nach oben als auch von oben nach unten durchfahren werden. Zur Erreichung einer guten Verteilung des Ausgangsproduktes über den gesamten Reaktorquerschnitt und zur Erzielung einer optimalen Berührung mit dem sauren Kontakt können die Apparate mit dem Fachmann bekannten Einbauten versehen werden, wie Düsen oder Lochplatten für den Eintritt des Ausgangsproduktes, sowie Böden und Umlenkbleche auf der gesamten Länge des Reaktors. Der Reaktor kann auch als Röhrenreaktor ausgebildet sein.

Bei der Anwendung eines kombinierten Reaktors für die Stufen a) und b) des erfindungsgemäßen Verfahrens wird das Ausgangsgemisch von unten oder von oben, je nach Art des verwendeten sauren Kontakts, in den Reaktor eingebracht. An einer geeigneten Stelle des kombinierten Reaktors, an der, in Fließrichtung des Reaktionsgemisches gesehen, 50 bis 90 Gew.-% des im Ausgangsprodukt vorhandenen iso-Butens zu iso-Buten-Oligomeren umgesetzt sind, wird sodann Methanol in der weiter oben beschriebenen Menge eingespeist und die Reaktion mit der Stufe b) des erfindungsgemäßen Verfahrens zu Ende gebracht.

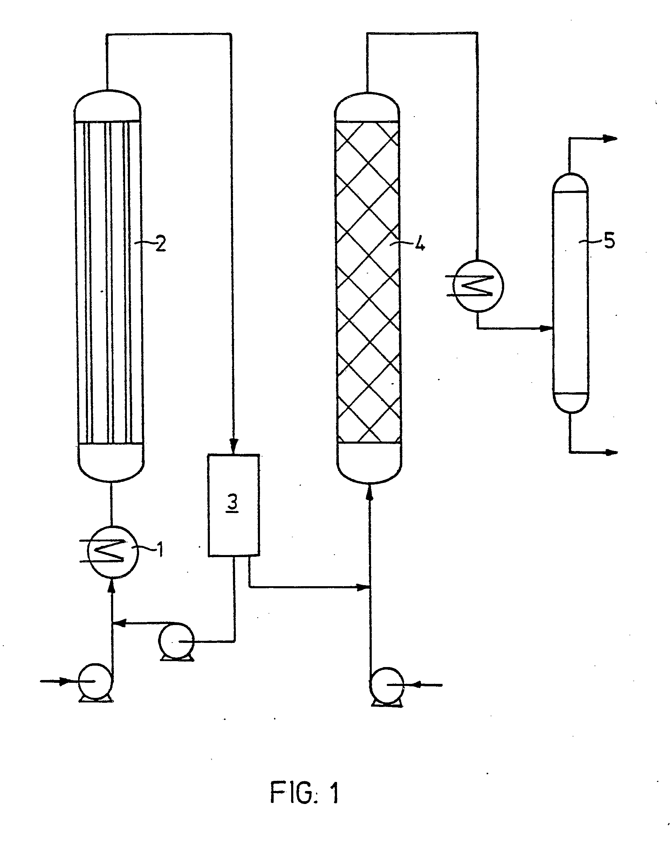

In einer anderen Verfahrensvariante werden die Stufen a) und b) des erfindungsgemäßen Verfahrens in getrennten Reaktionsapparaten durchgeführt. Hierbei können die Reaktionsapparate für beide Stufen des Verfahrens verschieden ausgeführt werden, beispielsweise als Röhrenreaktor oder als Festbettreaktor. Ferner können in den Reaktoren für die beiden Stufen der gleiche saure Kontakt oder verschiedene saure Kontakte von den obengenannten verwendet werden. Eine solche Verfahrensvariante sei anhand der Fig. 1 beschrieben :

Das iso-Buten-haltige $C_4$-Gemisch wird mit Hilfe einer Pumpe über einen Vorwärmer (1) in einen Festbettröhrenreaktor (2) eingebracht. Der Reaktor (2) besteht aus einem gekühlten Röhrensystem, dessen Röhren mit dem gewählten Katalysator gefüllt sind und dessen Freiraum um die Röhren herum mit einer geeigneten Kühlflüssigkeit zur Abführung der exothermen Reaktionswärme befahren werden kann. Die Pumpe hält einen solchen Druck aufrecht, daß das Reaktionsgemisch flüssig bleibt. Das Reaktionsgemisch wird aus dem Reaktor (2) zunächst in ein Zwischengefäß (3) geführt, von dem aus ein Teil des Gemisches als Kreislaufstrom in den Reaktor (2) zurückgeführt werden kann. Der restliche Anteil des Gemisches aus dem Zwischengefäß (3) wird in einen Festbettreaktor (4) geführt und vor Eintritt in diesen Reaktor mit der erfindungsgemäß erforderlichen Menge Methanol versetzt. Auch der Reaktor (4) wird unter einem solchen Druck betrieben, daß das Reaktionsgemisch flüssig bleibt. Nach Verlassen des Reaktors (4) wird das Reaktionsgemisch in eine Trennapparatur (Debutanizer (5)) eingebracht und in das erfindungsgemäß hergestellte Treibstoffgemisch als Sumpfprodukt und das $C_4$-Raffinat als Kopfprodukt getrennt.

Im erfindungsgemäßen Verfahren kann ein Gemisch von Diisobuten, Triisobuten, Tetraisobuten und Methyl-tert.-butyl-ether hergestellt werden, das nur noch einen untergeordneten Anteil an $C_4$-Kohlenwasserstoffen enthält und das, gegebenenfalls nach vollständiger oder teilweiser Hydrierung der olefinischen Anteile, sowohl als fertiger Motortreibstoff, aber auch als Zusatz zu anderen Motortreibstoffen zur Erhöhung von deren Oktanzahl verwendet werden kann.

Die Erfindung betrifft daher die Verwendung des erfindungsgemäß hergestellten Gemisches als Treibstoff oder als Zusatz zu einem Treibstoff für Vergasermotoren.

Die Erfindung betrifft weiterhin Treibstoffe für Vergasermotoren, die das erfindungsgemäß hergestellte Gemisch enthalten.

Selbstverständlich ist es auch möglich, das im erfindungsgemäßen Verfahren herstellbare Gemisch in die einzelnen reinen Komponenten aufzutrennen und diese gesondert als Zusätze zu Motortreibstoffen oder als Lösungsmittel zu verwenden.

Die Oligomerisierung des iso-Butens im erfindungsgemäßen Verfahren führt hauptsächlich zum 2,4,4-Trimethylpenten-(1), dessen hohe Oktanzahl bekannt ist.

Durch die Kreislauffahrweise und die Temperatureinstellung der Oligomerisierung in der Stufe a) des erfindungsgemäßen Verfahrens kann der Anteil an $C_8$-, $C_{12}$- und $C_{16}$-Oligomeren den Anforderungen an den Motortreibstoff angepaßt werden. Eine Rückführung des in der abschließenden Trennung im erfindungsgemäßen Verfahren anfallenden $C_4$-Kohlenwasserstoffstromes, wie in anderen Verfahren beschrieben, ist nicht erforderlich, da das iso-Buten selektiv praktisch vollständig umgesetzt wird. Dieser anfallende $C_4$-Strom aus der Trennstufe des erfindungsgemäßen enthält etwa 25 bis 40 Gew.-% wertvolles n-Buten-1.

In der Stufe b) des erfindungsgemäßen Verfahrens wird das nicht umgesetzte iso-Buten mit Methanol je nach Fahrweise dieser Stufe so umgesetzt, daß das Endprodukt etwa 5 bis etwa 40 Gew.-% Methyl-tert.-butyl-ether enthält. Da dieser Ether bekanntlich eine Testoktanzahl von 117 hat, wird das bereits hochoktanige Oligomerisat nochmals verbessert.

Überraschenderweise wird die Bildung von Dimethylhexenen beispielsweise aus iso-Buten und n-Butenen mit der niedrigen Testoktanzahl von etwa 93 weitgehend zurückgedrängt.

Im Gegensatz zu Verfahren des Standes der Technik, die lange Verweilzeiten zur weitgehenden Umsetzung des i-Butens erfordern, erlaubt das erfindungsgemäße Verfahren kürzere Verweilzeiten, wodurch die unerwünschte Bildung höherer Oligomerer zurückgedrängt wird und auch keine nennenswerte Isomerisierung des wertvollen n-Buten-1 zu n-Buten-2 stattfindet.

Weiterhin entfallen beim erfindungsgemäßen Verfahren aufwendige Trennoperationen zur Entfernung großer Überschüsse von Methanol von nach der Herstellung von Methyl-tert.-butyl-ether verbliebenen $C_4$-Kohlenwasserstoffen. Entsprechend dem Stande der Technik müssen solche $C_4$-Kohlenwasserstoffe für nachfolgende Alkylierungsreaktionen sogar durch eine Wasserwäsche vom restlichen Methanol befreit werden.

Die Erfindung wird an nachstehenden Beispielen näher erläutert, wobei eine Apparatur nach Fig. 1 verwendet wird.

## Beispiel 1

Von einem $C_4$-Strom eines Steam-Crackers wurden 2 820 g zunächst der Oligomerisierung und dann der Veretherung zugeführt. Der $C_4$-Schnitt hatte folgende Zusammensetzung :

| | |
|---|---|
| i-Butan | 2 Vol.-% |
| n-Butan | 8 Vol.-% |
| i-Buten | 47 Vol.-% |
| n-Buten-1 | 27 Vol.-% |
| Buten-2 | 16 Vol.-% |

Die Oligomerisierung wurde bei einem Druck von 17 bar und einer Reaktionstemperatur von 51 °C durchgeführt.

Als Kontakt wurde das sulfonsäurehaltige, vernetzte Polystyrol Lewatit SPC 118 eingesetzt. Nach Umsetzung von 75 % des eingesetzten i-Butens zu Diisobuten wurden 230 g Methanol zugesetzt. Die Raum-Stundengeschwindigkeit betrug 0,81 l $C_4$-Kohlenwasserstoffe/l Kontaktvolumen und Stunde.

Die Gesamtdauer des Versuches betrug 15 Stunden.

Nach der Veretherung wurde das Produkt in eine Destillationskolonne eingebracht und die $C_4$-Kohlenwasserstoffe vom übrigen Reaktionsgemisch abgetrennt. Das $C_4$-Raffinat wurde mit 1 245 g zurückgewonnen und betrug 40,8 Gew.-% aller eingesetzten Stoffe.

Die Analyse des Raffinats war folgende :

| | |
|---|---|
| i-Butan | 8,4 Gew.-% |
| n-Butan | 24,6 Gew.-% |
| i-Buten | 0,8 Gew.-% |
| n-Buten-1 | 38,1 Gew.-% |
| Buten-2 | 28,1 Gew.-% |

Aus dem Sumpf der Kolonne wurden 1 805 g abgezogen mit folgender Zusammensetzung :

| | |
|---|---|
| $C_4$-KWS* | < 1 Gew.-% |
| $C_8$-KWS | 50 Gew.-% |

| | |
|---|---|
| C$_{12}$-KWS | 19 Gew.-% |
| C$_{16}$-KWS | 2 Gew.-% |
| MTBE | 27 Gew.-% |
| Methanol | 2 Gew.-% |

Bromzahl (gBr$_2$/100 ml) = 100

* Kohlenwasserstoffe

Der Anteil des Reaktionsproduktes beträgt 59,2 Gew-% vom Einsatz.
Als motorische Daten werden folgende Werte ermittelt :

| | | |
|---|---|---|
| ROZ | clear | 102,2 |
| ROZ | 0,15 TEL/l | 103,2 |
| MOZ | clear | 83,6 |
| MOZ | 0,15 TEL/l | 85,7 |

Nach einer anschließenden partiellen Hydrierung am handelsüblichen Palladiumkatalysator wurde eine Bromzahl von ca. 40 eingestellt und folgende motorische Daten erhalten :

| | | |
|---|---|---|
| ROZ | clear | 101,9 |
| ROZ | 0,15 TEL/l | 102,9 |
| MOZ | clear | 87,3 |
| MOZ | 0,15 TEL/l | 90,2 |

## Beispiel 2

In einem zweiten Versuch wurde ein gleiches Reaktorsystem mit dem sauren, sulfonsäuregruppenhaltigen Ionenaustauscher Amberlyst 15 gefüllt.

Im Gegensatz zum Beispiel 1 wird eine dreifache Menge von Oligomerisat als Kreislauf gefahren. Dadurch konnte die Raum/Stundengeschwindigkeit bedeutend erhöht werden.

Sie betrug im vorliegenden Beispiel, bezogen auf C$_4$-Einsatz, 4,5 l C$_4$-Kohlenwasserstoff/l Kontaktvolumen und Stunde.

Es wurde wieder ein C$_4$-Strom eines Steam-Crackers nach der Butadien-Entfernung eingesetzt mit folgender Zusammensetzung :

| | |
|---|---|
| i-Butan | 2 Gew.-% |
| n-Butan | 8 Gew.-% |
| i-Buten | 47 Gew.-% |
| n-Buten-1 | 27 Gew.-% |
| Buten-2 | 16 Gew.-% |

Die Oligomerisierung wurde in einem Röhrenreaktor bei 51,2 °C durchgeführt. Aus einem Zwischengefäß wurde nach der Oligomerisierung und vor der Veretherung ein Kreislaufstrom abgenommen, der der dreifachen Menge des C$_4$-Kohlenwasserstoff Einsatzes entspricht, um das Einsatzprodukt zu verdünnen.

Die Veretherung des Rest-i-Butens im Reaktionsgemisch wurde ebenfalls am Amberlyst 15 durchgeführt.

Die Raum/Stundengeschwindigkeit betrug, bezogen auf den C$_4$-Einsatz, 4,5. Der Druck im System betrug konstant 17 bar.

Der Versuch dauerte 11 Stunden und 40 Minuten.

In dieser Zeit wurden 3 190 g C$_4$-Kohlenwasserstoffe eingefahren. Nach der Oligomerisierung von 76 % des i-Butens wurden in der Zeit 195 g Methanol eingefahren. Die Reaktionstemperatur im Veretherungsreaktor betrug 63 °C.

Nach der Reaktion wurde das Produkt in eine Kolonne gefahren und das C$_4$-Gemisch als Raffinat vom übrigen Reaktionsprodukt abgetrennt. Das C$_4$-Raffinat wurde zu 1 430 g ermittelt und betrug somit 42,2 Gew.-% vom Gesamteinsatz.

Die Analyse war folgende :

| | |
|---|---|
| i Butan | 8,0 Gew.-% |
| n-Butan | 25,0 Gew.-% |

6

| | |
|---|---|
| i-Buten | 0,5 Gew.-% |
| n-Buten-1 | 40,0 Gew.-% |
| Buten-2 | 26,5 Gew.-% |

Aus dem Sumpf der Kolonne wurden 1 955 g eines Produktes mit folgender Zusammensetzung abgezogen :

| | |
|---|---|
| $C_4$-KWS | < 1 Gew.-% |
| $C_8$-KWS | 57 Gew.-% |
| $C_{12}$-KWS | 14 Gew.-% |
| $C_{16}$-KWS | 2 Gew.-% |
| MTBE | 26 Gew.-% |
| Methanol | 1 Gew.-% |
| Bromzahl ($gBr_2$/100 ml) = 100 | |

Der Anteil des Reaktionsproduktes beträgt 57,8 % vom Gesamteinsatz. Als motorische Daten wurden folgende Zahlen ermittelt :

| | | |
|---|---|---|
| ROZ | clear | 102,2 |
| ROZ | 0,15 TEL/l | 103,1 |
| MOZ | clear | 85,4 |
| MOZ | 0,15 TEL/l | 86,8 |

## Beispiel 3

Aus einem bestehenden System von fünf Schleifenreaktoren wird nach dem dritten Reaktor das Reaktionsprodukt abgezogen und das nicht umgesetzte i-Buten am Amberlyst 15 mit Methanol verethert. Es wurde ein Kohlenwasserstoffgemisch aus dem dritten Schleifenreaktor mit folgender Zusammensetzung eingesetzt :

| | |
|---|---|
| $C_4$-KWS | 44,5 Gew.-% |
| $C_8$-KWS | 35,2 Gew.-% |
| $C_{12}$-KWS | 18,9 Gew.-% |
| $C_{16}$-KWS | 1,4 Gew.-% |

In den $C_4$-Kohlenwasserstoffen waren noch 9,5 % i-Buten enthalten, was einer Menge von 19,3 %, bezogen auf den ursprünglichen Einsatz, entspricht. 3 100 g dieses Produktes wurden mit 185 g Methanol, (1,1-Faches der molaren Menge, bezogen auf i-Buten), versetzt.

Die Veretherung wurde bei einer Temperatur von 63,5 °C und einem Druck von 17 bar durchgeführt.

Die Raum/Stundengeschwindigkeit während des Versuchs betrug 4,1 l $C_4$-Kohlenwasserstoff Einsatz/l Kontaktvolumen und Stunde.

Nach der Reaktion wurden in einer Destillationskolonne die $C_4$-Kohlenwasserstoffe vom übrigen Reaktionsprodukt abgetrennt.

Das $C_4$-Raffinat hatte folgende Zusammensetzung :

| | |
|---|---|
| i-Butan | 4,4 Gew.-% |
| n-Butan | 16,3 Gew.-% |
| i-Buten | 0,6 Gew.-% |
| n-Buten-1 | 38,9 Gew.-% |
| Buten-2 | 39,8 Gew.-% |

Das flüssige Reaktionsprodukt hatte nach der $C_4$-Abtrennung folgende Zusammensetzung :

| | |
|---|---|
| $C_4$-KWS | < 1 Gew.-% |
| $C_8$-KWS | 48,5 Gew.-% |
| $C_{12}$-KWS | 25,6 Gew.-% |
| $C_{16}$-KWS | 2,1 Gew.-% |
| MTBE | 21,8 Gew.-% |
| Methanol | 1 Gew.-% |

Die Menge wurde zu 2 200 g ermittelt und betrug somit 67,0 % vom Gesamteinsatz.

7

**0 028 377**

Ansprüche

1. Verfahren zur Herstellung eines Gemisches, das im wesentlichen aus iso-Buten-Oligomeren und Methyl-tert.-butyl-ether besteht, dadurch gekennzeichnet, daß man

a) ein Gemisch der geradkettigen und verzweigten Butane und Butene an einem sauren Kontakt so umsetzt, daß 50 bis 90 Gew.-% des in dem Gemisch enthaltenen iso-Butens in iso-Buten-Oligomere umgewandelt werden,

b) das so erhaltene Umsetzungsgemisch anschließend mit einer mindestens molaren Menge Methanol, bezogen auf das restliche iso-Buten, an einem sauren Kontakt umsetzt und

c) die nicht umgesetzten Butane und Butene vom Reaktionsgemisch abtrennt.

2. Verfahren nach Anspruch 1, dadurch gekennzeichnet, daß als saurer Kontakt für die Verfahrensstufen a) und b) stark saure Ionenaustauscher eingesetzt werden.

3. Verfahren nach Anspruch 1 und 2, dadurch gekennzeichnet, daß als saurer Kontakt ein sulfonsäuregruppenhaltiger Ionenaustauscher auf der Basis eines durch Divinylbenzol vernetzten Polystyrols eingesetzt wird.

4. Verfahren nach Anspruch 1 bis 3, dadurch gekennzeichnet, daß die Verfahrensstufe a) bei einer Temperatur von 20 bis 120 °C durchgeführt wird.

5. Verfahren nach Anspruch 1 bis 4, dadurch gekennzeichnet, daß a) und b) in getrennten Apparaten durchgeführt werden und ein Teil des Reaktionsgemisches der Stufe a) im Kreislauf durch den Reaktor für die Stufe a) gefahren wird.

6. Verfahren nach Ansprüchen 1 bis 3, dadurch gekennzeichnet, daß die Verfahrensstufe b) bei einer Temperatur von 25 bis 90 °C durchgeführt wird.

7. Verfahren nach Anspruch 1 bis 5, dadurch gekennzeichnet, daß die Stufen a) und b) unter einem solchen Druck durchgeführt werden, daß sich das gesamte Reaktionsgemisch im flüssigen Aggregatzustand befindet.

8. Verfahren nach Ansprüchen 1 bis 6, dadurch gekennzeichnet, daß die im Verfahren nach Anspruch 1 hergestellten, im Gemisch enthaltenen Olefine ganz oder teilweise hydriert werden.

9. Verwendung des im Verfahren nach Anspruch 1 bis 8 hergestellten Gemisches, das im wesentlichen aus gegebenenfalls ganz oder teilweise hydrierten iso-Buten-Oligomeren und Methyl-tert.-butyl-ether besteht, als Treibstoff oder als Zusatz zu einem Treibstoff für Vergasermotoren.

10. Treibstoff für Vergasermotoren, enthaltend das im Verfahren nach Anspruch 1 bis 8 hergestellte Gemisch, das im wesentlichen aus gegebenenfalls ganz oder teilweise hydrierten iso-Buten-Oligomeren und Methyl-tert.-butyl-ether besteht.

**Claims**

1. Process for the preparation of a mixture consisting essentially of iso-butene oligomers and methyl tert.-butyl ether, characterised in that

a) a mixture of straight-chain and branched butanes and butenes are reacted on an acid catalyst in a manner such that 50 to 90 % by weight of the iso-butene contained in the mixture is converted into iso-butene oligomers,

b) the reaction mixture thus obtained is then reacted with at least a molar amount of methanol, relative to the residual iso-butene, on an acid catalyst and

c) the unreacted butanes and butenes are separated off from the reaction mixture.

2. Process according to Claim 1, characterised in that strongly acid ion exchangers are employed as the acid catalyst for process stages a) and b).

3. Process according to Claims 1 and 2, characterised in that an ion exchanger which contains sulphonic acid groups and is based on a polystyrene crosslinked by divinylbenzene is employed as the acid catalyst.

4. Process according to Claims 1 to 3, characterised in that process stage a) is carried out at a temperature of 20 to 120 °C.

5. Process according to Claims 1 to 4, characterised in that a) and b) are carried out in separate apparatuses and some of the reaction mixture from stage a) is passed in circulation through the reactor for stage a).

6. Process according to Claims 1 to 3, characterised in that process stage b) is carried out at a temperature of 25 to 90 °C.

7. Process according to Claims 1 to 5, characterised in that stages a) and b) are carried out under a pressure such that the entire reaction mixture is in the liquid state of aggregation.

8. Process according to Claims 1 to 6, characterised in that the olefines prepared in the process according to Claim 1 and contained in the mixture are completely or partly hydrogenated.

9. Use of the mixture which is prepared in the process according to Claims 1 to 8 and consists essentially of optionally completely or partly hydrogenated iso-butene oligomers and methyl tert.-butyl ether, as a fuel or as an additive to a fuel for carburettor-type engines.

10. Fuel for carburettor-type engines containing the mixture which is prepared in the process

8

according to Claims 1 to 8 and consists essentially of optionally completely or partly hydrogenated iso-butene oligomers and methyl tert.-butyl ether.

## Revendications

1. Procédé de préparation d'un mélange constitué essentiellement d'oligomères d'isobutène et d'éther méthyl-tert-butylique, caractérisé en ce que :

a) on fait réagir un mélange des butènes et des butanes à chaînes droites et ramifiées sur un catalyseur acide de façon à transformer 50 à 90 % en poids de l'isobutène contenu dans le mélange en oligomères d'isobutène,

b) on fait ensuite réagir le mélange réactionnel ainsi obtenu avec une quantité au moins molaire de méthanol (rapporté à l'isobutène résiduel) sur un catalyseur acide, et

c) on sépare les butanes et les butènes n'ayant pas réagi du mélange réactionnel.

2. Procédé suivant la revendication 1, caractérisé en ce que, comme catalyseur acide pour les étapes opératoires a) et b), on utilise des échangeurs d'ions fortement acides.

3. Procédé suivant les revendications 1 et 2, caractérisé en ce que, comme catalyseur acide, on utilise un échangeur d'ions contenant des groupes d'acides sulfoniques et à base d'un polystyrène réticulé par le divinylbenzène.

4. Procédé suivant les revendications 1 à 3, caractérisé en ce qu'on effectue l'étape opératoire a) à une température de 20 à 120 °C.

5. Procédé suivant les revendications 1 à 4, caractérisé en ce qu'on effectue les étapes opératoires a) et b) dans des appareils séparés, tandis que l'on fait passer une partie du mélange réactionnel de l'étape a) dans le circuit à travers le réacteur prévu pour l'étape a).

6. Procédé suivant les revendications 1 à 3, caractérisé en ce qu'on effectue l'étape opératoire b) à une température de 25 à 90 °C.

7. Procédé suivant les revendications 1 à 5, caractérisé en ce qu'on effectue les étapes a) et b) sous une pression calculée de telle sorte que tout le mélange réactionnel soit à l'état d'agrégat liquide.

8. Procédé suivant les revendications 1 à 6, caractérisé en ce qu'on hydrogène entièrement ou partiellement les oléfines contenues dans le mélange et préparées par le procédé suivant la revendication 1.

9. Utilisation du mélange préparé dans le procédé suivant les revendications 1 à 8, ce mélange étant constitué essentiellement d'éther méthyl-tert-butylique et d'oligomères d'isobutène éventuellement hydrogénés entièrement ou partiellement, comme carburant ou comme additif à un carburant pour moteurs à carburateur.

10. Carburant pour moteurs à carburateur contenant le mélange préparé par le procédé suivant les revendications 1 à 8, ce mélange étant constitué essentiellement d'éther méthyl-tert-butylique et d'oligomères d'isobutène éventuellement hydrogénés entièrement ou partiellement.

FIG: 1